(19) Europäisches Patentamt
European Patent Office
Office européen des brevets

(11) EP 3 479 767 A1

(12) EUROPEAN PATENT APPLICATION

(43) Date of publication:
08.05.2019 Bulletin 2019/19

(51) Int Cl.:
$A61B\ 5/11$ (2006.01)    $A61B\ 5/024$ (2006.01)
$A61B\ 5/08$ (2006.01)    $A61B\ 5/1455$ (2006.01)
$A61B\ 5/113$ (2006.01)    $G06T\ 7/20$ (2017.01)

(21) Application number: 17199819.8

(22) Date of filing: 03.11.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventors:
• TEN KATE, Warner Rudolph Theophile
5656 AE Eindhoven (NL)
• KIRENKO, Ihor Olehovych
5656 AE Eindhoven (NL)
• HARMA, Aki Sakari
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) DISTANCE MEASUREMENT DEVICES, SYSTEMS AND METHODS, PARTICULARLY FOR USE IN VITAL SIGNS MEASUREMENTS

(57) The present invention relates to a distance measurement device and method for measuring the distance between an imaging unit and a subject carrying a sensor. The device comprises a sensor input (32) configured to obtain a sensor signal from the sensor carried by the subject, said sensor signal allowing deriving a length of movement of the subject between two points in time, and an image input (33) configured to obtain image data from the imaging unit, said image data depicting the positions of the subject at said two points in time. A length determination unit (34) determines the length of movement of the subject from said sensor signal between said two points in time, a displacement unit (35) determines the displacement of the subject between the positions of the subject at said two points in time from said image data, and a distance determination unit (38) determines the distance between the imaging unit and the subject from the determined length of movement and the determined displacement.

FIG.3

EP 3 479 767 A1

**Description**

FIELD OF THE INVENTION

The present invention relates to distance measurement devices, systems and methods for measuring the distance between an imaging unit and a subject, particularly for use in vital signs measurements. Further, the present invention relates to a vital signs determination device for determining / measuring one or more vital signs of a subject.

BACKGROUND OF THE INVENTION

Imaging units, such as cameras, provide effective and powerful means to monitor a subject's health status, e.g. of a person or patient, such as an elderly person in a care home, a patient in a hospital, a premature baby, etc. By placing a camera to view the subject's bed, vital signs like heart rate, respiration rate, SpO2, etc. can be measured by use of the know technology called remote photo-plethysmography (PPG) without the need to attach sensors to the subject. When the subject is mobile and can leave the bed, additional wearable sensors can be complementary.

The camera can also be used to monitor and detect other aspects of the care needed. For example, the camera can be used to detect if the patient is leaving the bed or has fallen off the bed.

When using a 2D camera the actual distance of the patient to the camera cannot be measured and will remain unknown. This unknown may hamper other usages of the camera in which the observed (pixel) changes in the 2D plane are used to monitor the subject's movements. Outcomes might be less useful or accurate without knowledge of the distance of the subject from the camera. Without information about the distance of the subject the excursion of the movement cannot be estimated. For example, knowing the distance enables to estimate the movement of the chest or a blanket of a subject, and determine the subject or a distance of a movement of a subject or other object. Moreover, 2D data would not allow robust and easy detection of occlusion situations, or reliable tracking of several subjects simultaneously.

It is possible to get a distance estimate using 3D or depth camera techniques. However, the price of depth-sensing camera technologies, their installation and calibration, and the data transmission required for 3D data makes them non-practical in many environments. Moreover, the need to have a dedicated 3D front end (e.g. a time-of-flight (ToF) sensor) to provide robust monitoring hampers the introduction of that monitoring technology in systems, which already use installed 2D cameras.

SUMMARY OF THE INVENTION

It is an object of the present invention to provide distance measurement devices, systems and methods that allow measuring the distance between an imaging unit and a subject without much additional hard- and/or software, particularly for a particular camera or for installation and/or calibration.

It is a further object of the present invention to provide a vital signs determination unit that allows measuring vital signs more reliably and accurately.

In a first aspect of the present invention a distance measurement device is presented comprising:

- a sensor input configured to obtain a sensor signal from the sensor carried by the subject, said sensor signal allowing deriving a length of movement of the subject between two points in time,
- an image input configured to obtain image data from the imaging unit, said image data depicting the positions of the subject at said two points in time,
- a length determination unit configured to determine the length of movement of the subject from said sensor signal between said two points in time,
- a displacement unit configured to determine the displacement of the subject between the positions of the subject at said two points in time from said image data, and
- a distance determination unit configured to determine the distance between the imaging unit and the subject from the determined length of movement and the determined displacement.

In a further aspect of the present invention a distance measurement system is presented comprising:

- a sensor configured to acquire a sensor signal while the sensor is carried by the subject, said sensor signal allowing deriving a length of movement of the subject between two points in time,
- an imaging unit configured to acquire image data, said image data depicting the positions of the subject at said two points in time,
- a distance measurement device as disclosed herein for determining the distance between the imaging unit and the subject carrying a sensor based on the acquired sensor signal and the acquired image data.

In a further aspect of the present invention a vital signs determination unit is presented comprising:

- a distance measurement device as disclosed herein, and
- a vital signs determination unit configured to determine a vital sign of the subject from said image data and the distance determined by said distance measurement device.

In yet further aspects of the present invention, there are provided corresponding distance measurement methods, a computer program which comprises program code means for causing a computer to perform the steps of the methods disclosed herein when said computer program is carried out on a computer as well as a non-transitory computer-readable recording medium that stores therein a computer program product, which, when executed by a processor, causes the methods disclosed herein to be performed.

Preferred embodiments of the invention are defined in the dependent claims. It shall be understood that the claimed methods, system, computer program and medium have similar and/or identical preferred embodiments as the claimed devices, in particular as defined in the dependent claims and as disclosed herein.

The present invention is based on the idea to make use of one or more sensor signals of one or more sensors carried by the subject. Such a sensor may be worn by the subject, e.g. a patient in a hospital, anyhow for other purposes. For instance, an accelerometer (ACC) sensor may be attached to the patient's chest to monitor chest movements caused by breathing to monitor if the patient is breathing and optionally to determine the respiration rate. A Wi-Fi communication unit may be carried by the subject, e.g. as part of a body area network to collect and transmit signals of various sensors attached to the patient's body to e.g. a patient monitor, or as part of a user device (e.g. a smartphone or smart watch).

In order to estimate distance, the displacements by the subject worn sensor(s) are computed, and these are correlated with those in the imaging unit (e.g. a camera), i.e. the displacement in number of pixels. Using their ratio, or, alternatively, the size of their correlation coefficient, the distance from the imaging unit is computed by applying the geometry of the setup.

It should be noted that the length of movement of the subject and the displacement of the subject can be determined continuously, regularly or only at discrete times. The subject needs not to move continuously between the two points in time between which both the length of movement and the displacement are determined from the two different kinds of data input to the position determination device. The two points in time may be selected arbitrarily, but should be selected such that a sufficiently large movement exists between said two points in time enabling the determination of a length of movement and of a displacement of the subject.

Typically, movements in the range of centimeters, e.g. in the range of 1 cm to 50 cm, particularly between 5 cm and 25 cm, deliver good results. The time period (i.e. between the first point in time and the second point in time) for which said length of movement is determined may in the range of seconds or even smaller, e.g. in the range of 0.1 s to 10 s, particularly between 0.5 s and 3 s.

In an embodiment said length determination unit is configured to determine the length of movement of the subject projected into or within a common plane, in particular a common plane that is substantially perpendicular to the optical path of the imaging unit from the imaging unit to the subject. This increases accuracy of the measurement. To a certain extent also for movements of the subject in the direction of the imaging unit the desired distance can be determined. For instance, once a correlation between length of movement and displacement has been established and the size of the subject has been determined, the distance can be estimated from the apparent size of the subject in the image data.

Hereby, said length determination unit is preferably configured to determine the length of movement of the subject in vertical and/or horizontal direction. This is particularly used if the sensor provides sensor measurements in vertical and/or horizontal direction, like e.g. an accelerometer.

In another embodiment said sensor input is configured to obtain a sensor signal from a pressure sensor, GPS sensor, Wi-Fi communication unit, radio signal communication unit and/or ultrasound sensor and said length determination unit is configured to determine the length of movement of the subject between said two points in time from said sensor signal by determining height changes and/or horizontal movements of the sensor. For instance, a pressure sensor provides information on height changes. A GPS sensor can provide 2D or 3D position information. A Wi-Fi communication unit, e.g. a Wi-Fi receiver, and a radio signal communication unit, e.g. a mobile communication unit for mobile communication, can also be used to provide a position information. An ultrasound sensor can determine the distance to objects in the surroundings, e.g. floor and/or ceiling. A change in this distance provides the information of the length of movement.

According to an embodiment said sensor input is configured to obtain an accelerometer signal from an accelerometer and said length determination unit is configured to determine the length of movement of the subject from said accelerometer signal by determining height changes and/or horizontal movements by double integrating the accelerometer signal. Such accelerometer sensors are often used in a clinical context for respiration monitoring of a patient and can thus be easily used without much additional effort in the context of the present invention.

Hereby, said length determination unit may be configured to integrate the spatial components of the accelerometer signal separately for two or more spatial coordinates, which further improves accuracy of the distance determination.

Further, the length determination unit may be configured in this context to apply a common rotation or normalization to the integrated accelerometer signals. The signals can be transformed from the sensor's coordinate system (since the sensor can be in any orientation) to the coordinates in global space (in which the imaging

**EP 3 479 767 A1**

unit measures 'up' and 'down'). This transformation can be expressed by a rotation matrix. The accelerometer signal generally comprises three components, for the sensor's x, y, and z axis. This makes a sample of this signal a vector. In the global coordinate system other x, y and z axes are used, and the vector values change to represent the same vector in space. This change may be done by multiplying the vector in sensor coordinates with the rotation matrix (yielding the vector in global coordinates).

In an embodiment said distance determination unit is configured to determine the distance between the imaging unit and the subject from the determined length of movement and the determined displacement and from a calibration factor representing the ratio between a known length of movement of the subject and a corresponding known displacement. Such a calibration factor may be obtained in advance, e.g. by calibration measurements and e.g. use of a calibration object, or as a learning system from actual measurements over time.

Said length determination unit may further be configured to determine the length of movement of the subject and/or said distance determination unit may further be configured to determine the distance between the imaging unit and the subject only if the image data indicate that the subject has moved. This saves computation power and time. At times where no (or insufficient, i.e. too small) movement is detected, no further computations for determining length of movement and/or determining displacement and/or determining final distance may thus be performed.

In still another aspect of the present invention another distance measurement device is presented comprising:

- a sensor input configured to obtain an accelerometer signal from an accelerometer carried by the subject between two points in time,
- an image input configured to obtain image data from the imaging unit, said image data depicting the positions of the subject at said two points in time,
- a displacement unit configured to determine the displacement of the subject at said two points in time from said image data, and
- a distance determination unit configured to determine the distance between the imaging unit and the subject by i) double differentiating the determined displacement and comparing it with the accelerometer signal at the positions of the subject at said two points in time or ii) by once differentiating the determined displacement and once integrating the accelerometer signal and comparing them.

This aspect is generally based on the same idea than the other aspects discussed above. Rather than using a distance that is derived from an accelerometer signal, e.g. by double integration, a velocity and/or accelerometer signal may be determined and used, requiring a single or double differentiation of the determined displacement enabling a comparison between a signal derived from the sensor signal and a signal derived from the image data. However, also in this aspect a sensor signal from a sensor worn by the subject is used for correlation with a signal derived from the image data. As with distance, the comparison is identical: it is about establishing a ratio between the two quantities, which ratio is proportional to the distance. The ratio is the same for acceleration, velocity and displacement (and any other derivative), since they are linearly related. The advantage is that better signal-noise ratios may be obtained, or that the computation is more efficient. Differentiation at the imaging unit might be easier supported by power constraints than integration at the wireless sensor. Preferably the comparison is in the displacement domain (integration has a low-pass, i.e. averaging effect, and hence, in general, reduces noise level).

## BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a first embodiment of a distance measurement system according to the present invention,
Fig. 2 shows a schematic diagram of a first embodiment of a distance measurement device according to the present invention,
Fig. 3 shows a diagram illustrating general geometry used according to the present invention, and
Fig. 4 shows a schematic diagram of a second embodiment of a distance measurement device according to the present invention.
Fig. 5 shows a schematic diagram of a vital signs determination device according to an embodiment of the invention.

## DETAILED DESCRIPTION OF THE EMBODIMENTS

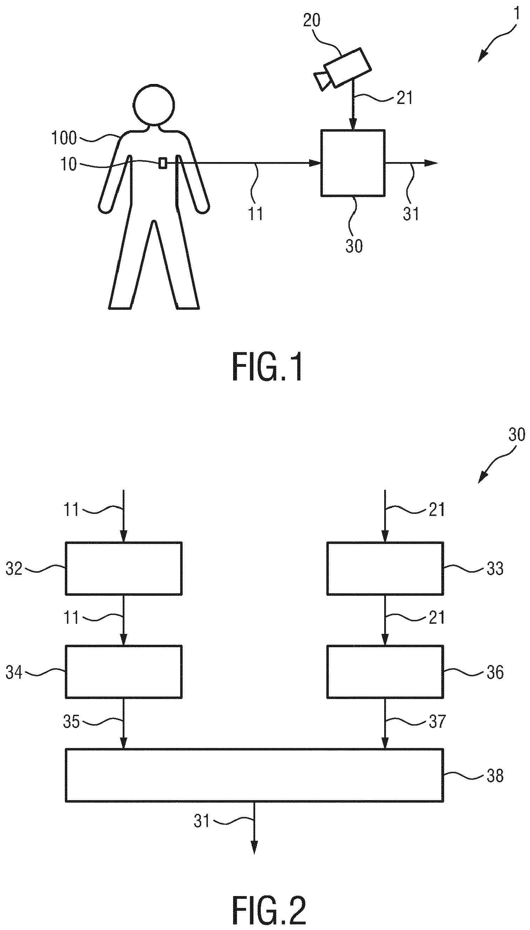

Fig. 1 shows a schematic diagram of a first embodiment of a distance measurement system 1 according to the present invention. The distance measurement system 1 comprises a sensor 10 configured to acquire a sensor signal 11 while the sensor 10 is carried by the subject 10, e.g. a patient walking around in a hospital. The sensor 10 is of such a kind that the sensor signal 11 allows deriving a length of movement of the subject 100 between two points in time, e.g. a time duration of less than a second up to a few seconds while the subject 100 is e.g. walking or rising up.

Examples of the sensor 10 are an accelerator, a pressure sensor, a GPS sensor, a Wi-Fi communication unit, a radio signal communication unit and/or an ultrasound sensor. The sensor 10 may be carried by the subject 10 in any possible way, e.g. attached to the body (e.g. by use of a strap or belt or sticker), or integrated into a user device such as a smartphone or smart watch, or integrated into clothing worn by the subject 100, or even implanted into a part of the subject's body.

The distance measurement system 1 further comprises an imaging unit 20, e.g. a camera such as an RGB camera, configured to acquire image data 21. The image data 21 depict the positions of the subject 100 at said two points in time, at which the length of movement will be derived from the sensor signal 11. The image data 21 may be continuous video data or may be image frames taken at discrete moments in time, e.g. every 100 ms or every second. The imaging unit 20 may, in the above mentioned exemplary application where the subject 100 is a patient walking through a hospital, one of a plurality of cameras mounted at different locations at the ceiling or wall of the hospital to monitor the hospital and any movements.

The distance measurement system 1 further comprises a distance measurement device 30 as disclosed herein for determining the distance 31 between the imaging unit 20 and the subject 100 carrying a sensor 10. The distance 31 is determined based on the acquired sensor signal 11 and the acquired image data 21. The distance measurement device 30 may be implemented in hard- and/or software, e.g. as a programmed processor or computer, and may be integrated into the imaging unit 20, particularly if the imaging unit is also configured (or provided with means) for determining one or more vital signs of the subject 100 from the image data 21 using remote PPG technology. Using PPG technology, vital signs can be measured, which are revealed by minute light absorption changes in the skin caused by the pulsating blood volume, i.e. by periodic color changes of the human skin induced by the blood volume pulse. This is e.g. described in Verkruysse et al., "Remote plethysmographic imaging using ambient light", Optics Express, 16(26), 22 December 2008, pp. 21434-21445.

Fig. 2 shows a schematic diagram of a first embodiment of a distance measurement device 30 according to the present invention. The distance measurement device 30 comprises a sensor input 32 configured to obtain the sensor signal 11 from the sensor 10 carried by the subject 100 and an image input 33 configured to obtain image data 21 from the imaging unit 20. The sensor input 32 and the image input 33 may be configured as wireless or wired data interfaces, e.g. as Bluetooth interface, Wi-Fi interface, USB interface, computer network interface, mobile communication interface, etc., for obtaining (i.e. retrieving or receiving) the respective data either directly from the sensor 10 or imaging unit 20, respectively, or indirectly via a buffer or storage unit where these data are buffered or stored. The sensor input 32 and the image input 33 maybe configured as separate interfaces or as a common interface that is coupled with the sensor 10 and the imaging unit 20.

The distance measurement device 30 further comprises a length determination unit 34 configured to determine the length 35 of movement of the subject 100 from said sensor signal 11 between said two points in time, at which the image data depict the position of the movement of the subject 100. Hereby, the length determination unit 34 determines the distance (i.e. the length of movement) between the position of the subject 100 at a first point in time and the position of the subject 100 at a second point in time. This measurement preferably determines the length of movement of the subject projected into or within a common plane, in particular a common plane that is substantially perpendicular to the optical path of the imaging unit 20 from the imaging unit 20 to the subject 100. Particularly movements in horizontal and/or vertical direction are measured.

The distance measurement device 30 further comprises a displacement unit 36 configured to determine the displacement 37 of the subject 100 between the positions of the subject 100 at said two points in time from said image data 21. The displacement unit 36 e.g. determines the number of pixels between the position of the subject 100 at the first point in time as reflected in an image frame acquired at the first point in time and the second point in time as reflected in an image frame acquired at the second point in time.

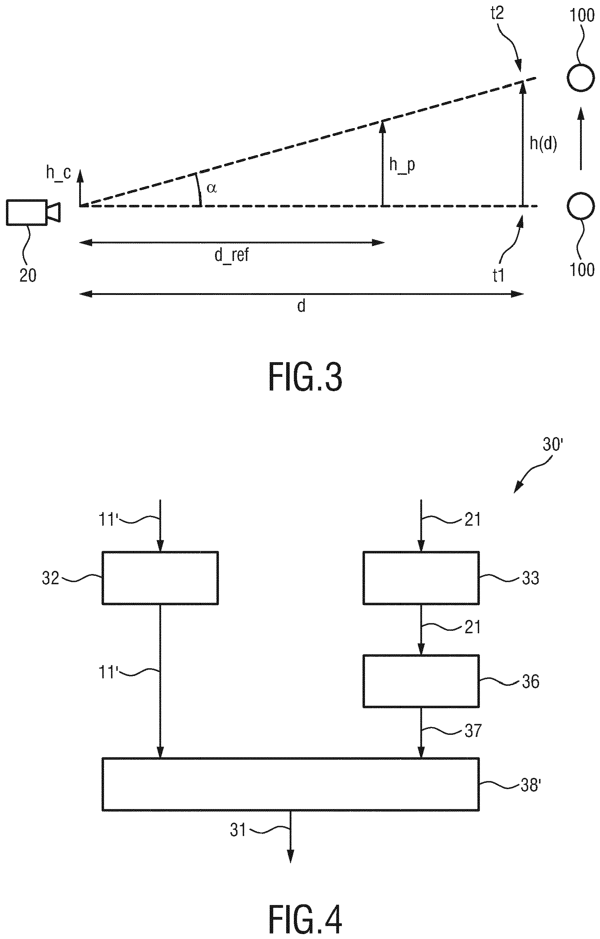

The distance measurement device 30 further comprises a distance determination unit 38 configured to determine the distance 31 between the imaging unit 20 and the subject 100 from the determined length 35 of movement and the determined displacement 37. This shall be illustrated by the diagram shown in Fig. 3 illustrating general geometry used according to the present invention.

In Fig. 3 a camera 20, at the left side, is viewing a patient 100, at the right side. The patient 100 is carrying a sensor 10 (not shown) or a sensing system (not shown) comprising such a sensor. When the patient 100 moves, a length of movement h(d) is made in a time interval between a first point in time t1 and a second point in time t2. This length of movement h(d) appears at the camera 20 (i.e. within the image data, particularly from a combination of at least two images taken at the first point in time t1 and the second point in time t2) as a displacement h_c. Their ratio (h(d) / h_c) is proportional to the actual distance d of the patient 100 to the camera 20. This ratio also scales with the viewing angle $\alpha$ (or numerical aperture) of the camera 20.

Preferably, only the length of movement in a common plane, particularly a plane perpendicular to the line connecting camera 20 to patient 100, or the length of movement projected in that plane are observed. In general, the height changes fulfil this condition. An additional correction factor can be included to account for camera viewing directions that are not so aligned. The correction factor performs the projection on the perpendicular plane.

For example, the camera may have an accelerometer and/or magnetometer from which its viewing direction can be determined. In case the algorithm selects vertical movements, an accelerometer in the camera tells the viewing angle relative to the horizontal plane, from which the correction angle can be determined: if the accelerometer measures gravity as vector (x, y, z) in the sensor's coordinates (assumed to be aligned with the camera's viewing direction), the angle with the horizontal follows from the dot product of this (x, y, z) with the vertical (0, 0, 1): $\alpha$ = a cos(z). The x and y components may additionally be used for refined estimation.

Since the ratio indicates the distance in a proportional manner, it only informs about the relative distance (to a previous situation). Since the above mentioned correction factor (for perpendicular projection) is constant, it does not affect the relative distance and hence is not relevant in this case.

When estimating the absolute distance the camera 100 is preferably calibrated, where, next to the above mentioned correction factor, the viewing angle $\alpha$ (or numerical aperture (NA)) of the camera 20 is a parameter. The viewing angle changes when the focus (numerical aperture) of the camera changes, but can be determined from the actual focus setting of the camera. Zooming also scales the number of pixels of the images.

For instance, the viewing angle can be determined from a calibration step. A subject could move in front of the camera and create a table that directly relates the measured ratio to actual distance (so that NA effects are included) (this is the parameter c_0 explained below). For varying NA (zooming) the changing zoom is known and used. In case of digital zoom, the number of pixels for given displacement (and distance) stays the same, so the computation is straightforward.

At the reference distance *d_ref,* used for the calibration, a height change *h_p* of the patient corresponds to a pixel change *h_c* in the images according to

$$(1) \quad h\_c = c\_0 \cdot h\_p$$

where *c_0* is a constant (including the above mentioned correction factor). By conducting the calibration measurement this constant *c_0* can be determined.

The height change *h(d)* at another distance *d,* i.e. if the patient movement results in a length of movement *h(d),* corresponds to *h_p* (at *d_ref),* i.e. results in the same height change *h_c* at the camera, according to

$$(2) \quad h\_p = h(d) \cdot d\_ref \,/\, d$$

Combining (1) and (2) and solving for d yields

$$(3) \quad d = (c\_0 \cdot d\_ref) \cdot h(d) \,/\, h\_c$$

The factor (*c_0 · d_ref*) is found during calibration (for the respective focus settings). *h(d)* and *h_c* are measured during operation, from the worn sensor and camera pixels, respectively.

As explained above, different kinds of sensors 10 may be used for acquiring the sensor signal 11. For instance, an air pressure sensor may be used that enables to measure height changes, since height change is (negatively) proportional to the product of environmental pressure and pressure change. An accelerometer also enables to measure height changes, and in addition, at least to some extent, horizontal displacements. In an embodiment the acceleration signal is double integrated, but further measures may need to be performed. Example measures that may be taken are to filter the accelerometer signal with non-linear filters (to obtain the gravity component in the acceleration), opposed to linear low-pass filtering, and to apply the filter step again after the first integration to velocity. Further measures may e.g. be found in EP 2745120 A1.

In practical situations, using e.g. the moment the patient is rising (from the bed or a chair) is a favorable movement that is evaluated since it involves a vertical displacement. Vertical displacements can be estimated most accurate using one or more accelerometers. Also air pressure sensors can then be used (they only respond to vertical displacement). Bed rise and chair rise can be detected from the body sensor(s) as well as the imaging unit. Such rise takes typically a time in the order of one second, which in general is also beneficial for the displacement estimation. The displacement estimation can even be optimized for this time window (and vertical movement). The distance during walking can subsequently be estimated from the image data alone using the vertical movements for re-calibration (fusion) of the distance.

As illustrated above, in order to estimate the desired distance, the length of movement by the user worn sensor(s) is computed, and this is correlated with the displacement reflected in the images acquired by the camera, i.e. the displacement in number of pixels. Using their ratio, or, alternatively, the size of the correlation coefficient, the distance from the camera is computed by applying the geometry of the setup.

In embodiments the geometric distortions of the lenses of the camera in different parts of the field of view are taken into account to calibrate the distance estimate. In the above description the geometric correction of the optical setup is omitted. For instance, the optical aberration in the image may give an inaccurate measure of the true movements of the object in the field of view. Therefore, it maybe advisable to compensate for the errors caused by geometric distortions. The correction of an image for optical distortions is known in the area of machine vision and the theory and methodology is well understood. In an embodiment the correction is performed by first correcting the image geometry before computation of the displacements. Alternatively, the correction can be performed for the measured displacement or by manipulation of the estimate of the movement to match with the known image distortion.

In the above the displacements are compared. When the body worn sensor is an accelerometer, an alternative scheme is to (double) differentiate the displacement h_c in the camera and to compare that (its ratio or its correlation) with the acceleration at the patient. Obviously, other schemes are also conceivable, such as comparing (correlating) velocity. This might be beneficial when a velocity related sensor is used, such as an air flow sensor. Fig. 4 shows a schematic diagram of a second embodiment of a distance measurement device 30' according to the present invention making use of this approach.

The distance measurement device 30' comprises the sensor input 32 that obtains an accelerometer signal 11' (as sensor signal) from an accelerometer (as sensor 10) carried by the subject between two points in time. The distance measurement device 30' comprises the image input 33 that obtains image data 21 from the imaging unit, said image data depicting the positions of the subject at said two points in time. The distance measurement device 30' comprises the displacement unit 36 that determines the displacement 37 of the subject at said two points in time from said image data 21.

Different from the distance measurement device 30 the distance measurement device 30' does not comprise a length determination unit and comprises a different distance determination unit 38' that determines the distance 31 between the imaging unit 21 and the subject 100 either i) by double differentiating the determined displacement 37 and comparing it with the accelerometer signal 11' at the positions of the subject at said two points in time or ii) by once differentiating the determined displacement 37 and once integrating the accelerometer signal 11' and comparing them.

In the estimation of the velocity or a distance from the accelerometer signal 11' it is integrated over time once or twice, respectively, to the target measure (i.e. velocity or length of movement). The integration can be performed for each spatial coordinate separately or followed by a common rotation or a normalization operation applied to the vector-valued measurements of the accelerometer signal 11'. The integration is very sensitive to noise and non-linear distortions in the measurement data which often leads to a drift of the values.

In another embodiment the image data is used in the estimation of the length of movement h(d) so that the accelerometer signal is only integrated in time segments when there is a motion detected in the image plane from the image data.

In another embodiment, in certain scenes with static objects, the distance defined based on the abode described analysis of the sensor signal of e.g. a wearable accelerometer sensor, can be associated with the static objects in the scene, which may serve as landmarks of the distance for 3D reconstruction of the scene.

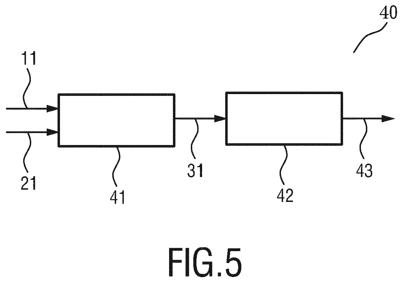

The present invention can generally be applied in all scenarios where a sensor is carried by a subject, who is monitored by a camera, in order to determine the distance between the subject and the camera. A preferred application is in the field of vital signs measurement using remote PPG technology. A schematic diagram of a vital signs determination device 2 is depicted in Fig. 5. Said vital signs determination device 40 comprises a distance measurement device 41 (e.g. a distance measurement device 30, 30' as disclosed herein) and a vital signs determination unit 42 that is configured to determine a vital sign 43 of the subject from said image data 21 and the distance 31 between the imaging unit 20 and the subject 100 determined by said distance measurement device 41.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Distance measurement device for measuring the distance between an imaging unit and a subject carrying a sensor, said device comprising:

> - a sensor input (32) configured to obtain a sensor signal from the sensor carried by the subject, said sensor signal allowing deriving a length of movement of the subject between two points in time,
> - an image input (33) configured to obtain image data from the imaging unit, said image data depicting the positions of the subject at said two points in time,
> - a length determination unit (34) configured to determine the length of movement of the subject from said sensor signal between said two points in time,
- a displacement unit (35) configured to determine the displacement of the subject between the positions of the subject at said two points in time from said image data, and
- a distance determination unit (38) configured to determine the distance between the imaging unit and the subject from the determined length of movement and the determined displacement.

2. Device as claimed in claim 1,
wherein said length determination unit (34) is configured to determine the length of movement of the subject projected into or within a common plane, in particular a common plane that is substantially perpendicular to the optical path of the imaging unit from the imaging unit to the subject.

3. Device as claimed in claim 2,
wherein said length determination unit (34) is configured to determine the length of movement of the subject in vertical and/or horizontal direction.

4. Device as claimed in claim 1,
wherein said sensor input (32) is configured to obtain a sensor signal from a pressure sensor, GPS sensor, Wi-Fi communication unit, radio signal communication unit and/or ultrasound sensor and said length determination unit () is configured to determine the length of movement of the subject between said two points in time from said sensor signal by determining height changes and/or horizontal movements of the sensor.

5. Device as claimed in claim 1,
wherein said sensor input (32) is configured to obtain an accelerometer signal from an accelerometer and said length determination unit (34) is configured to determine the length of movement of the subject from said accelerometer signal by determining height changes and/or horizontal movements by double integrating the accelerometer signal.

6. Device as claimed in claim 5,
wherein said length determination unit (34) is configured to integrate the spatial components of the accelerometer signal separately for two or more spatial coordinates.

7. Device as claimed in claim 6,
wherein said length determination unit (34) is configured to apply a common rotation or normalization to the integrated accelerometer signals.

8. Device as claimed in claim 1,
wherein said distance determination unit (3 8) is configured to determine the distance between the imaging unit and the subject from the determined length of movement and the determined displacement and from a calibration factor representing the ratio between a known length of movement of the subject and a corresponding known displacement.

9. Device as claimed in claim 1,
wherein said length determination unit (34) is configured to determine the length of movement of the subject and/or said distance determination unit (38) is configured to determine the distance between the imaging unit and the subject only if the image data indicate that the subject has moved.

10. Distance measurement device for measuring the distance between an imaging unit and a subject carrying an accelerometer, said device comprising:

- a sensor input (32) configured to obtain an accelerometer signal from an accelerometer carried by the subject between two points in time,
- an image input (33) configured to obtain image data from the imaging unit, said image data depicting the positions of the subject at said two points in time,
- a displacement unit (36) configured to determine the displacement of the subject at said two points in time from said image data, and
- a distance determination unit (38') configured to determine the distance between the imaging unit and the subject by i) double differentiating the determined displacement and comparing it with the accelerometer signal at the positions of the subject at said two points in time or ii) by once differentiating the determined displacement and once integrating the accelerometer signal and comparing them.

11. Vital signs determination device comprising:

- a distance measurement device (41) as claimed in claim 1 or 10, and
- a vital signs determination unit (42) configured to determine a vital sign of the subject from said image data and the distance determined by said distance measurement device.

12. Distance measurement system for measuring the distance between an imaging unit and a subject carrying a sensor, said system comprising:

- a sensor (10) configured to acquire a sensor signal while the sensor is carried by the subject, said sensor signal allowing deriving a length of movement of the subject between two points in time,
- an imaging unit (20) configured to acquire image data, said image data depicting the positions of the subject at said two points in time,
- a distance measurement device (30, 30') as claimed in claim 1 or 10 for determining the distance between the imaging unit and the subject carrying a sensor based on the acquired sensor signal and the acquired image data.

13. Distance measurement method for measuring the distance between an imaging unit and a subject carrying a sensor, said method comprising:

- obtaining a sensor signal from the sensor carried by the subject, said sensor signal allowing deriving a length of movement of the subject between two points in time,
- obtaining image data from the imaging unit, said image data depicting the positions of the subject at said two points in time,
- determining the length of movement of the subject from said sensor signal between said two points in time,
- determining the displacement of the subject between the positions of the subject at said two points in time from said image data, and
- determining the distance between the imaging unit and the subject from the determined length of movement and the determined displacement.

14. Distance measurement method for measuring the distance between an imaging unit and a subject carrying an accelerometer, said method comprising:

- obtaining an accelerometer signal from an accelerometer carried by the subject between two points in time,
- obtaining image data from the imaging unit, said image data depicting the positions of the subject at said two points in time,
- determining the displacement of the subject between the positions of the subject at said two points in time from said image data, and
- determining the distance between the imaging unit and the subject by i) double differentiating the determined displacement and comparing it with the accelerometer signal at the positions of the subject at said two points in time or ii) by once differentiating the determined displacement and the accelerometer signal and comparing them.

15. Computer program comprising program code means for causing a computer to carry out the steps of the method as claimed in claim 13 or 14 when said computer program is carried out on the computer.

FIG.1

FIG.2

FIG.3

FIG.4

40

11

21 41 31 42 43

FIG.5

Europäisches
Patentamt

European
Patent Office

Office européen
des brevets

## EUROPEAN SEARCH REPORT

Application Number

EP 17 19 9819

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Jun Kawai ET AL: "IDENTIFICATION AND POSITIONING BASED ON MOTION SENSORS AND A VIDEO CAMERA", Proc. 4th IASTED Int. Conf. on Web-Based Education , 1 January 2005 (2005-01-01), XP55478334, Retrieved from the Internet: URL:https://pdfs.semanticscholar.org/0c15/afddb88958b04c92e21416e83f5bcf6753bd.pdf [retrieved on 2018-05-24] * Sections 1, 3.1, 3.2, 4.2 and 5; figures 1-7 * | 1-15 | INV. A61B5/11 ADD. A61B5/024 A61B5/08 A61B5/1455 A61B5/113 G06T7/20 |
| X | US 2015/154447 A1 (WILSON ANDREW D [US] ET AL) 4 June 2015 (2015-06-04) * paragraphs [0060] - [0062], [0073] - [0104]; figures 1-8 * | 1-15 | |
| A | EP 2 438 849 A1 (UNIV LATVIJAS [LV]) 11 April 2012 (2012-04-11) * paragraphs [0023] - [0033]; figures 1-3 * | 1-15 | TECHNICAL FIELDS SEARCHED (IPC) A61B G06T |

The present search report has been drawn up for all claims

| Place of search | Date of completion of the search | Examiner |
|---|---|---|
| The Hague | 5 June 2018 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 9819

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2018

| Patent document cited in search report | | | Publication date | Patent family member(s) | | | Publication date |
|---|---|---|---|---|---|---|---|
| US 2015154447 | A1 | | 04-06-2015 | CN<br>EP<br>US<br>US<br>WO | 105814609<br>3077992<br>2015154447<br>2017330031<br>2015084667 | A<br>A1<br>A1<br>A1<br>A1 | 27-07-2016<br>12-10-2016<br>04-06-2015<br>16-11-2017<br>11-06-2015 |
| EP 2438849 | A1 | | 11-04-2012 | EP<br>LV | 2438849<br>14514 | A1<br>B | 11-04-2012<br>20-08-2012 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2745120 A1

**Non-patent literature cited in the description**

- **VERKRUYSSE et al.** Remote plethysmographic imaging using ambient light. *Optics Express,* 22 December 2008, vol. 16 (26), 21434-21445